# EUROPEAN PATENT SPECIFICATION

(11) **EP 4 387 547 B1**
(45) Date of publication and mention of the grant of the patent: **28.05.2025**
(21) Application number: 22826541.9
(22) Date of filing: 08.11.2022
(51) Int. Cl.: A61B 18/12, A61B 18/14

(54) **SYSTEMS FOR ENERGIZING ELECTROPORATION CATHETERS USING QUADRIPOLAR ARRAYS**
SYSTEME ZUR ERREGUNG VON ELEKTROPORATIONSKATHETERN UNTER VERWENDUNG VIERPOLIGER ARRAYS
SYSTÈMES PERMETTANT D'ALIMENTER DES CATHÉTERS D'ÉLECTROPORATION À L'AIDE DE RÉSEAUX QUADRIPOLAIRES

(30) Priority: 12.11.2021 US 202163278605 P
(43) Date of publication of application: 26.06.2024
(73) Proprietor: St. Jude Medical, Cardiology Division, Inc., St. Paul, MN 55117 (US)
(72) Inventor: OLSON, Eric, Maplewood, Minnesota 55119 (US); ZHANG, Xiangyang, Maple Grove, Minnesota 55311 (US)
(74) Representative: Mathys & Squire
(86) International application number: PCT/US2022/079432
(87) International publication number: WO 2023/086778

(56) References cited:
- WO-A1-2018/201037
- US-A1- 2019 030 328
- US-A1- 2019 350 649
- US-A1- 2021 137 587
- US-A1- 2021 161 582

## Description

### FIELD OF THE DISCLOSURE

The present disclosure relates generally to tissue ablation systems. In particular, the present disclosure relates to applying electroporation therapy using a catheter including a plurality of electrodes defining at least one quadripolar array.

### BACKGROUND

It is generally known that ablation therapy may be used to treat various conditions afflicting the human anatomy. For example, ablation therapy may be used in the treatment of atrial arrhythmias. When tissue is ablated, or at least subjected to ablative energy generated by an ablation generator and delivered by an ablation catheter, lesions form in the tissue. Electrodes mounted on or in ablation catheters are used to create tissue necrosis in cardiac tissue to correct conditions such as atrial arrhythmia (including, but not limited to, ectopic atrial tachycardia, atrial fibrillation, and atrial flutter).

Arrhythmia (i.e., irregular heart rhythm) can create a variety of dangerous conditions including loss of synchronous atrioventricular contractions and stasis of blood flow which can lead to a variety of ailments and even death. It is believed that the primary cause of atrial arrhythmia is stray electrical signals within the left or right atrium of the heart. The ablation catheter imparts ablative energy (e.g., radiofrequency energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that lead to arrhythmias.

Electroporation is a non-thermal ablation technique that involves applying strong electric-fields that induce pore formation in the cellular membrane. The electric field may be induced by applying a relatively short duration pulse which may last, for instance, from a nanosecond to several milliseconds. Such a pulse may be repeated to form a pulse train. When such an electric field is applied to tissue in an in vivo setting, the cells in the tissue are subjected to trans-membrane potential, which opens the pores on the cell wall. Electroporation may be reversible (i.e., the temporally-opened pores will reseal) or irreversible (i.e., the pores will remain open). For example, in the field of gene therapy, reversible electroporation (i.e., temporarily open pores) is used to transfect high molecular weight therapeutic vectors into the cells. In other therapeutic applications, a suitably configured pulse train alone may be used to cause cell destruction, for instance by causing irreversible electroporation.

For example, pulsed field ablation (PFA) may be used to perform instantaneous pulmonary vein isolation (PVI). PFA generally involves delivering high voltage pulses from electrodes disposed on a catheter. For example, voltage pulses may range from less than about 500 volts to about 2400 volts or higher. These fields may be applied between pairs of electrodes (bipolar therapy) or between one or more electrodes and a return patch (monopolar therapy).

WO-A1-2018/201037 discloses systems, devices, and methods for delivery of pulsed electric field ablative energy to endocardial tissue. US-A1-2021/161582 discloses an electroporation system configured to apply a biphasic pulse signal to at least one electrode. US- A1-2019/030328 discloses expandable elements for delivery of electric fields.

### BRIEF SUMMARY OF THE DISCLOSURE

The invention is defined in independent calim 1. Preferred features are set out in the dependent claims. In one aspect, an apparatus for controlling an electroporation catheter is provided. The electroporation catheter includes a distal end, a proximal end, a plurality of splines extending from the distal end to the proximal end, and a plurality of electrodes arranged on the plurality of splines and defining at least one quadripolar array, each quadripolar array defined by four electrodes of the plurality of electrodes. The apparatus includes a pulse generator coupled to the electroporation catheter, and a computing device coupled to the pulse generator, the computing device operable to control the pulse generator to selectively energize the electrodes defining the at least one quadripolar array according to a first energization pattern, and selectively energize the electrodes defining the at least one quadripolar array according to a second energization pattern, wherein the first and second energization patterns are different from one another.

In another aspect, a method, which is not claimed as such, for controlling a system including an electroporation catheter, a pulse generator coupled to the electroporation catheter, and a computing device coupled to the pulse generator is provided. The electroporation catheter includes a distal end, a proximal end, a plurality of splines extending from the distal end to the proximal end, and a plurality of electrodes arranged on the plurality of splines and defining at least one quadripolar array, each quadripolar array defined by four electrodes of the plurality of electrodes. The method includes selectively energizing, using the computing device and the pulse generator, the electrodes defining the at least one quadripolar array according to a first energization pattern, and selectively energizing, using the computing device and the pulse generator, the electrodes defining the at least one quadripolar array according to a second energization pattern, wherein the first and second energization patterns are different from one another.

In yet another aspect, a system is provided. The system includes an electroporation catheter including a distal end, a proximal end, a plurality of splines extending from the distal end to the proximal end, and a plurality of electrodes arranged on the plurality of splines and defining at least one quadripolar array, each quadripolar array defined by four electrodes of the plurality of electrodes. The system further includes a pulse generator coupled to the electroporation catheter, and a computing device coupled to the pulse generator, the computing device operable to control the pulse generator to selectively energize the electrodes defining the at least one quadripolar array according to a first energization pattern, and selectively energize the electrodes defining the at least one quadripolar array according to a second energization pattern, wherein the first and second energization patterns are different from one another.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a schematic and block diagram view of a system for electroporation therapy.
Figure 2 is a side view of one embodiment of a grid assembly that may be used with the catheter shown in Figure 1.
Figure 3 is an image showing the grid assembly of Figure 2 positioned within a patient's heart.
Figures 4A-4C illustrate a plurality of example energization patterns using the grid assembly shown in Figure 2.
Figures 5A illustrates an example energization pattern using the grid assembly shown in Figure 2.
Figure 5B is a diagram simulating an electric field strength for the energization pattern shown in Figure 5A.
Figure 5C is a diagram simulating a potential field for the energization pattern shown in Figure 5A.
Figure 6 is a diagram simulating an electric field strength for an energization pattern that corresponds to the energization pattern Figure 4B.
Figure 7 is a diagram simulating an electric field strength for an energization pattern that corresponds to the energization pattern Figure 4C.
Figures 8A-8C are representations of the diagram shown in Figures 5B, 6, and 7.
Figure 9 is a diagram showing the representations of Figures 8A-8C superimposed on one another.
Figure 10A-10D illustrate a plurality of additional example energization patterns using the grid assembly shown in Figure 2.
Figures 11A and 11B are perspective views of one embodiment of a basket assembly that may be used with the catheter shown in Figure 1.
Figures 12A-12C are views of another embodiment of a basket assembly that may be used with the catheter shown in Figure 1.
Figure 13 is a schematic diagram of one embodiment of a switching architecture that may be used with the system shown in Figure 1.

### DETAILED DESCRIPTION OF THE DISCLOSURE

The systems and methods described herein are directed to an apparatus for controlling an electroporation catheter. The electroporation catheter includes a distal end, a proximal end, a plurality of splines extending from the distal end to the proximal end, and a plurality of electrodes arranged on the plurality of splines and defining at least one quadripolar array, each quadripolar array defined by four electrodes of the plurality of electrodes. The apparatus includes a pulse generator coupled to the electroporation catheter, and a computing device coupled to the pulse generator, the computing device operable to control the pulse generator to selectively energize the electrodes defining the at least one quadripolar array according to a first energization pattern, and selectively energize the electrodes defining the at least one quadripolar array according to a second energization pattern, wherein the first and second energization patterns are different from one another.

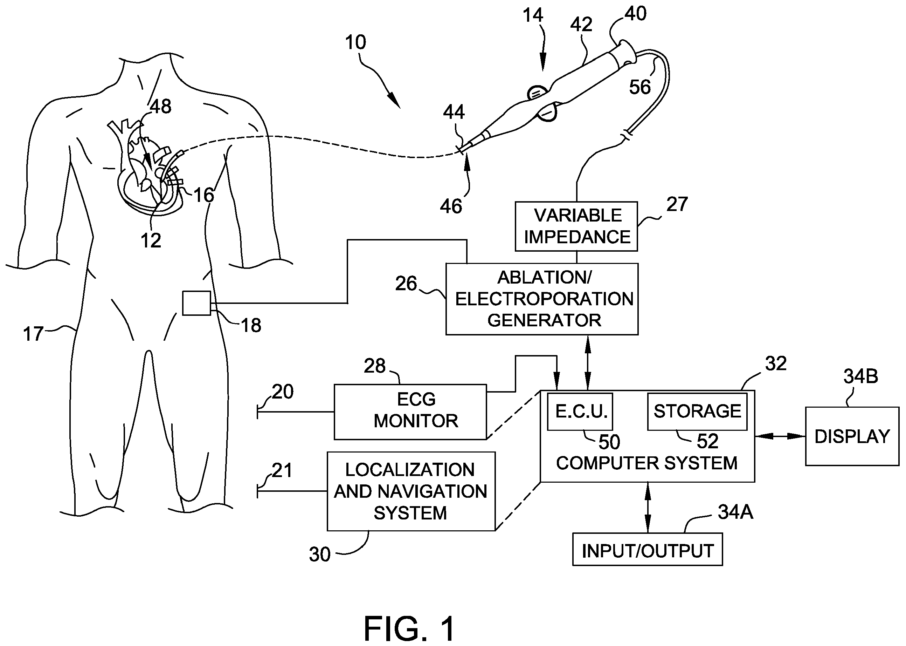

Figure 1 is a schematic and block diagram view of a system 10 for electroporation therapy. In general, system 10 includes a catheter electrode assembly 12 disposed at a distal end 48 of a catheter 14. As used herein, "proximal" refers to a direction toward the end of the catheter near the clinician and "distal" refers to a direction away from the clinician and (generally) inside the body of a patient. The electrode assembly includes one or more individual, electrically-isolated electrode elements. Each electrode element, also referred to herein as a catheter electrode, is individually wired such that it can be selectively paired or combined with any other electrode element to act as a bipolar or a multi-polar electrode.

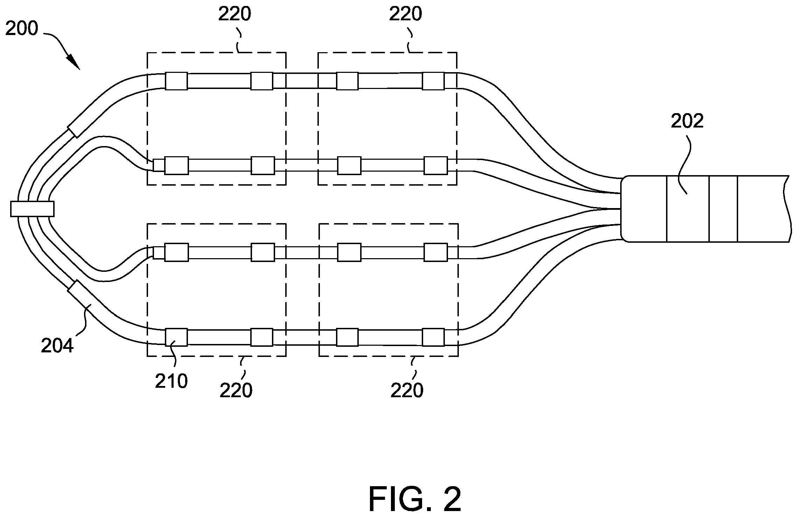

System 10 may be used for irreversible electroporation (IRE) to destroy tissue. In particular, system 10 may be used for electroporation-induced therapy that includes delivering electrical current in such a manner as to directly cause an irreversible loss of plasma membrane (cell wall) integrity leading to its breakdown and cell necrosis. This mechanism of cell death may be viewed as an "outside-in" process, meaning that the disruption of the outside wall of the cell causes detrimental effects to the inside of the cell. Typically, for classical plasma membrane electroporation, electric current is delivered as a pulsed electric field in the form of short-duration pulses (e.g., having a 100 nanosecond (ns) to 100 microsecond (µs) duration) between closely spaced electrodes capable of delivering an electric field strength of about 0.1 to 10.0 kilovolts/centimeter (kV/cm). System 10 may be used with a grid catheter such as that depicted in Figure 2, for example, for high output (e.g., high voltage and/or high current) electroporation procedures. Alternatively, system 10 may be used with any suitable catheter configuration.

In one embodiment, all electrodes of the catheter deliver an electric current simultaneously. Alternatively, in other embodiments, stimulation is delivered selectively (e.g., between pairs of electrodes) on the catheter. For example, in some embodiments, the catheter includes a plurality of splines, each spline including a plurality of electrodes. In such embodiments, electrodes on one spline may be selectively activated, and electrodes on an adjacent (or other) spline function as an energy return (or sink). Further, in the embodiments described herein, the electrodes may be switchable between being connected to a 3D mapping system and being connected to an electroporation generator.

Irreversible electroporation through a multi-electrode catheter may enable pulmonary vein isolation in as few as one shock per vein, which may produce much shorter procedure times compared to sequentially positioning a radiofrequency (RF) ablation tip around a vein.

It should be understood that while the energization strategies are described as involving DC pulses, embodiments may use variations. For example, exponentially-decaying pulses, exponentially- increasing pulses, and combinations may be used.

Further, it should be understood that the mechanism of cell destruction in electroporation is not primarily due to heating effects, but rather to cell membrane disruption through application of a high-voltage electric field. Thus, electroporation may avoid some possible thermal effects that may occur when using radio frequency (RF) energy. This "cold therapy" thus has desirable characteristics.

With this background, and now referring again to Figure 1, system 10 includes a catheter electrode assembly 12 including at least one catheter electrode. Electrode assembly 12 is incorporated as part of a medical device such as a catheter 14 for electroporation therapy of tissue 16 in a body 17 of a patient. In the illustrative embodiment, tissue 16 includes heart or cardiac tissue. It should be understood, however, that embodiments may be used to conduct electroporation therapy with respect to a variety of other body tissues.

Figure 1 further shows a plurality of return electrodes designated 18, 20, and 21, which are diagrammatic of the body connections that may be used by the various sub-systems included in overall system 10, such as an electroporation generator 26, an electrophysiology (EP) monitor such as an ECG monitor 28, and a localization and navigation system 30 for visualization, mapping, and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode, and may include split patch electrodes (as described herein). In other embodiments, return electrodes 18, 20, and 21 may be any other type of electrode suitable for use as a return electrode including, for example, one or more catheter electrodes. Return electrodes that are catheter electrodes may be part of electrode assembly 12 or part of a separate catheter or device (not shown). System 10 may further include a main computer system 32 (including an electronic control unit 50 and data storage-memory 52), which may be integrated with localization and navigation system 30 in certain embodiments. System 32 may further include conventional interface components, such as various user input/output mechanisms 34A and a display 34B, among other components.

Electroporation generator 26 is configured to energize the electrode element(s) in accordance with an electroporation energization strategy, which may be predetermined or may be user-selectable. For electroporation-induced primary necrosis therapy, generator 26 may be configured to produce an electric current that is delivered via electrode assembly 12 as a pulsed electric field in the form of short-duration DC pulses (e.g., a nanoseconds to several milliseconds duration, or any duration suitable for electroporation) between closely spaced electrodes capable of delivering an electric field strength (i.e., at the tissue site) of about 0.1 to 1.0 kV/cm. The amplitude and pulse duration needed for irreversible electroporation are inversely related.

Electroporation generator 26, sometimes also referred to herein as a DC energy source, is a biphasic electroporation generator 26 configured to generate a series of DC energy pulses that all produce current in two directions. In other embodiments, electroporation generator is a monophasic or polyphasic electroporation generator. In some embodiments, electroporation generator 26 is configured to output energy in DC pulses at selectable energy levels, such as fifty joules, one hundred joules, two hundred joules, and the like. Other embodiments may have more or fewer energy settings and the values of the available setting may be the same or different. For successful electroporation, some embodiments utilize the two hundred joule output level. For example, electroporation generator 26 may output a DC pulse having a peak magnitude from about 300 Volts (V) to about 3,200 V at the two hundred joule output level. Other embodiments may output any other suitable positive or negative voltage.

In some embodiments, a variable impedance 27 allows the impedance of system 10 to be varied to limit arcing. Moreover, variable impedance 27 may be used to change one or more characteristics, such as amplitude, duration, pulse shape, and the like, of an output of electroporation generator 26. Although illustrated as a separate component, variable impedance 27 may be incorporated in catheter 14 or generator 26.

With continued reference to Figure 1, as noted above, catheter 14 may include functionality for electroporation and in certain embodiments also additional ablation functions (e.g., RF ablation). It should be understood, however, that in those embodiments, variations are possible as to the type of ablation energy provided (e.g., cryoablation, ultrasound, etc.).

In the illustrative embodiment, catheter 14 includes a cable connector or interface 40, a handle 42, and a shaft 44 having a proximal end 46 and a distal 48 end. Catheter 14 may also include other conventional components not illustrated herein such as a temperature sensor, additional electrodes, and corresponding conductors or leads. Connector 40 provides mechanical and electrical connection(s) for cable 56 extending from generator 26. Connector 40 may include conventional components known in the art and as shown is disposed at the proximal end of catheter 14.

Handle 42 provides a location for the clinician to hold catheter 14 and may further provide means for steering or the guiding shaft 44 within body 17. For example, handle 42 may include means to change the length of a guidewire extending through catheter 14 to distal end 48 of shaft 44 or means to steer shaft 44. Moreover, in some embodiments, handle 42 may be configured to vary the shape, size, and/or orientation of a portion of the catheter, and it will be understood that the construction of handle 42 may vary. In an alternate embodiment, catheter 14 may be robotically driven or controlled. Accordingly, rather than a clinician manipulating a handle to advance/retract and/or steer or guide catheter 14 (and shaft 44 thereof in particular), a robot is used to manipulate catheter 14. Shaft 44 is an elongated, tubular, flexible member configured for movement within body 17. Shaft 44 is configured to support electrode assembly 12 as well as contain associated conductors, and possibly additional electronics used for signal processing or conditioning. Shaft 44 may also permit transport, delivery and/or removal of fluids (including irrigation fluids and bodily fluids), medicines, and/or surgical tools or instruments. Shaft 44 may be made from conventional materials such as polyurethane and defines one or more lumens configured to house and/or transport electrical conductors, fluids or surgical tools, as described herein. Shaft 44 may be introduced into a blood vessel or other structure within body 17 through a conventional introducer. Shaft 44 may then be advanced/retracted and/or steered or guided through body 17 to a desired location such as the site of tissue 16, including through the use of guidewires or other means known in the art.

In some embodiments, catheter 14 is a grid catheter having catheter electrodes (not shown in Figure 1) distributed at the distal end of shaft 44. In some embodiments, catheter 14 has sixteen catheter electrodes. In other embodiments, catheter 14 includes ten catheter electrodes, twenty catheter electrodes, or any other suitable number of electrodes for performing electroporation. In some embodiments, the catheter electrodes are ring electrodes, such as platinum ring electrodes. Alternatively, the catheter electrodes may be any other suitable type of electrodes, such as partial ring electrodes or electrodes printed on a flex material. In various embodiments, the catheter electrodes have lengths of 1.0 mm, 2.0 mm, 2.5 mm, and/or any other suitable length for electroporation.

Localization and navigation system 30 may be provided for visualization, mapping and navigation of internal body structures. Localization and navigation system 30 may include conventional apparatus known generally in the art. For example, localization and navigation system 30 may be substantially similar to the EnSite Precision^{™} System, commercially available from Abbott Laboratories, and as generally shown in commonly assigned U.S. Pat. No. 7,263,397 titled "Method and Apparatus for Catheter Navigation and Location and Mapping in the Heart". In another example, localization and navigation system 30 may be substantially similar to the EnSite X^{™} System, as generally shown in U.S. Pat. App. Pub. No. 2020/0138334 titled "Method for Medical Device Localization Based on Magnetic and Impedance Sensors". It should be understood, however, that localization and navigation system 30 is an example only, and is not limiting in nature. Other technologies for locating/navigating a catheter in space (and for visualization) are known, including for example, the CARTO navigation and location system ofBiosense Webster, Inc., the Rhythmia^{®} system of Boston Scientific Scimed, Inc., the KODEX^{®} system of Koninklijke Philips N.V., the AURORA^{®} system of Northern Digital Inc., commonly available fluoroscopy systems, or a magnetic location system such as the gMPS system from Mediguide Ltd. In this regard, some of the localization, navigation and/or visualization system would involve a sensor be provided for producing signals indicative of catheter location information, and may include, for example one or more electrodes in the case of an impedance-based localization system, or alternatively, one or more coils (i.e., wire windings) configured to detect one or more characteristics of a magnetic field, for example in the case of a magnetic-field based localization system As yet another example, system 10 may utilize a combination electric field-based and magnetic field-based system as generally shown with reference to U.S. Pat. No. 7,536,218 entitled "Hybrid Magnetic-Based and Impedance Based Position Sensing".

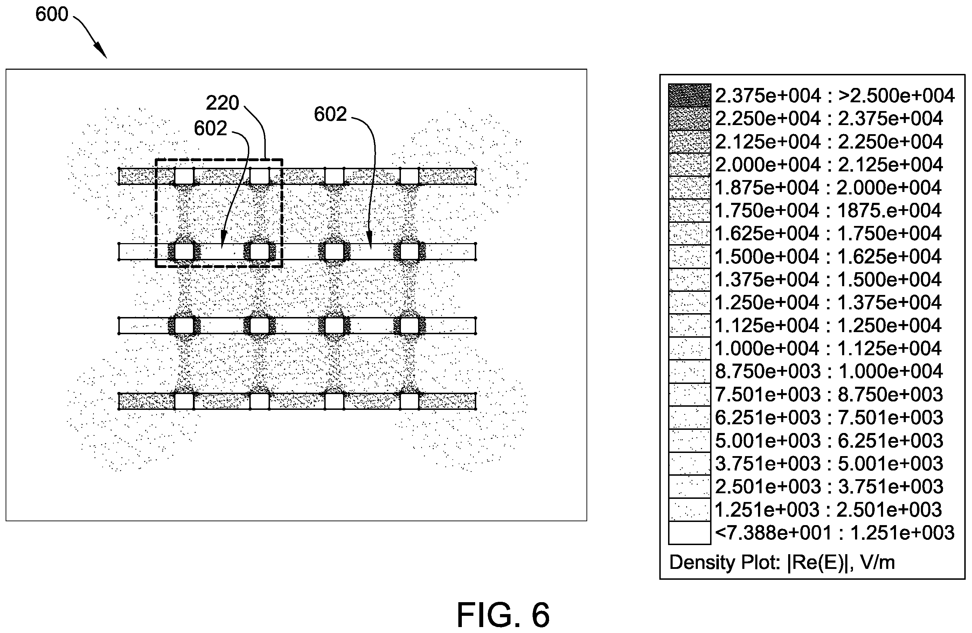

In at least some of the embodiments described herein, a catheter includes an array of electrodes that define one or more pixels. The array of electrodes may be arranged, for example, on a grid catheter (e.g., as shown in Figures 2-5B) or on a basket catheter (e.g., as shown in Figures 6A-7C). Alternatively, the array of electrodes may be arranged on any suitable catheter assembly.

Figure 2 is a side view of one embodiment of a grid assembly 200 that may be used with catheter 14 in system 10. Those of skill in the art will appreciate that, in other embodiments, any suitable catheter may be used. In addition, those of skill in the art will appreciate that, although the embodiments disclosed herein are discussed in the context of a grid catheter, the methods and systems described herein may be implemented using any suitable catheter (e.g., basket catheters, etc.). As shown in Figure 2, grid assembly 200 is coupled to a distal section 202 of shaft 44.

Grid assembly 200 includes a plurality of splines 204 extending from a proximal end 206 to a distal end 208. Each spline 204 includes a plurality of electrodes 210. In the embodiment shown in Figure 2, grid assembly 200 includes four splines 204, and each spline 204 includes four electrodes 210, such that electrodes 210 form a grid configuration. Accordingly, grid assembly 200 provides a four by four grid of electrodes 210. In one embodiment, the spacing between each pair of adjacent electrodes 210 is approximately 4 millimeters (mm) such that the dimensions of the grid of electrodes 210 are approximately 12 mm x 12 mm. Alternatively, grid assembly 200 may include any suitable number of splines 204, any suitable number of electrodes 210, and/or any suitable arrangement of electrodes 210. For example, in some embodiments, the spacing between each pair of adjacent electrodes is approximately 2 millimeters (mm). Further, in some embodiments, grid assembly 200 may include, for example, fifty-six electrodes arranged in a 7 x 8 grid.

Using grid assembly 200, lesions may be generated at individual electrodes 210 using a monopolar approach (e.g., by applying a voltage between individual electrodes 210 and a return patch), or generated between pairs of electrodes 210 using a bipolar approach. Lesions may be generating within an anatomy by selectively energizing electrodes in a particular configuration and/or pattern (e.g., including energizing individual electrodes 210 independent of one another, or energizing multiple electrodes 210 simultaneously).

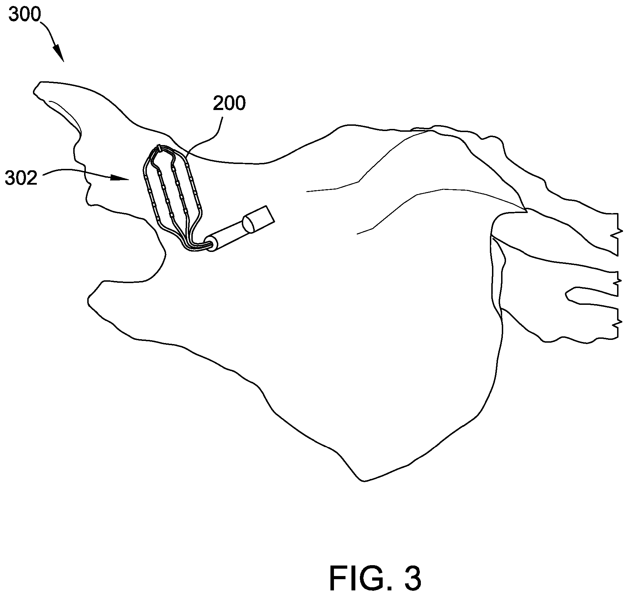

Figure 3 is an image 300 showing grid assembly 200 positioned within a left atrium 302 of a patient's heart. As shown in Figure 3, grid assembly 200 covers a relatively wide area of the heart. The width of this area is generally larger than that needed to perform pulmonary vein isolation (PVI). Accordingly, to perform a successful PVI ablation, it may be possible to only energize a portion of grid assembly 200.

Using bipolar delivery patterns, a plurality of different energization patterns are available using grid assembly 200. For example, each electrode 210 may selectively function as a positive electrode, a negative electrode, or an inactive electrode. If all electrodes 210 are energized at the same polarity, then an indifferent electrode (e.g., one of surface electrodes 18, 20, and 21 (shown in Figure 1)) functions as a return electrode. If some electrodes 210 are energized at a positive polarity, and other electrodes 210 are energized at a negative polarity, no indifferent electrode is required, as there are current paths between electrodes 210.

In the embodiments described herein, energy is delivered uniformly using quadripolar arrays (i.e., 2 x 2 arrays) of electrodes 210. In this embodiment, grid assembly 200 includes four quadripolar arrays 220, as shown in Figure 2. As will be appreciated by those of skill in the art, a plurality of different energization schemes are possible for a quadripolar array 220 of electrodes 210. For example, in some embodiments, different quadripolar arrays 220 may share at least one electrode 210.

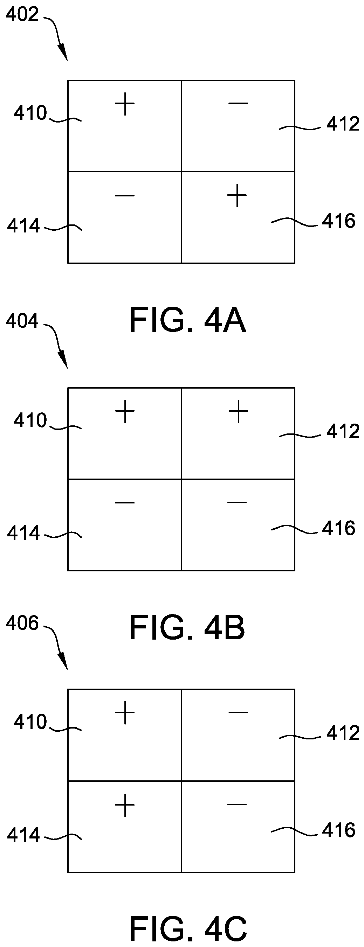

For example, Figures 4A, 4B, and 4C illustrate a first energization pattern 402, a second energization pattern 404, and a third energization pattern 406, respectively.

In first energization pattern 402, a first electrode 410 is positive, a second electrode 412 is negative, a third electrode 414 is negative, and a fourth electrode 416 is positive. In second energization pattern 404, first electrode 410 is positive, second electrode 412 is positive, third electrode 414 is negative, and fourth electrode 416 is negative. In third energization pattern 406, first electrode 410 is positive, second electrode 412 is negative, third electrode 414 is positive, and fourth electrode 416 is negative.

Those of skill in the art will appreciate that other energization patterns are possible. Notably, other energization patterns are redundant to those shown in Figures 4A-4C (i.e., with the polarity of each electrode 210 switched), degenerate (i.e., with all electrodes 210 having the same polarity), or unequal (i.e., having a different number of positive and negative electrodes 210).

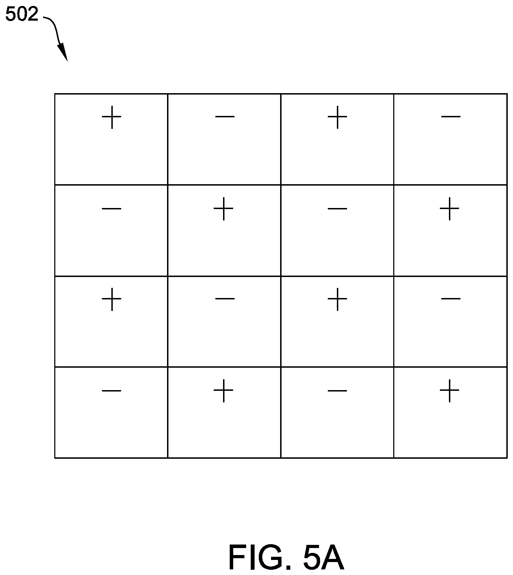

Figure 5A is an example energization pattern 502 for all sixteen electrodes 210 of catheter assembly 200. Specifically, energization pattern 502 corresponds to each quadripolar array 220 using first energization pattern 402 (shown in Figure 4A).

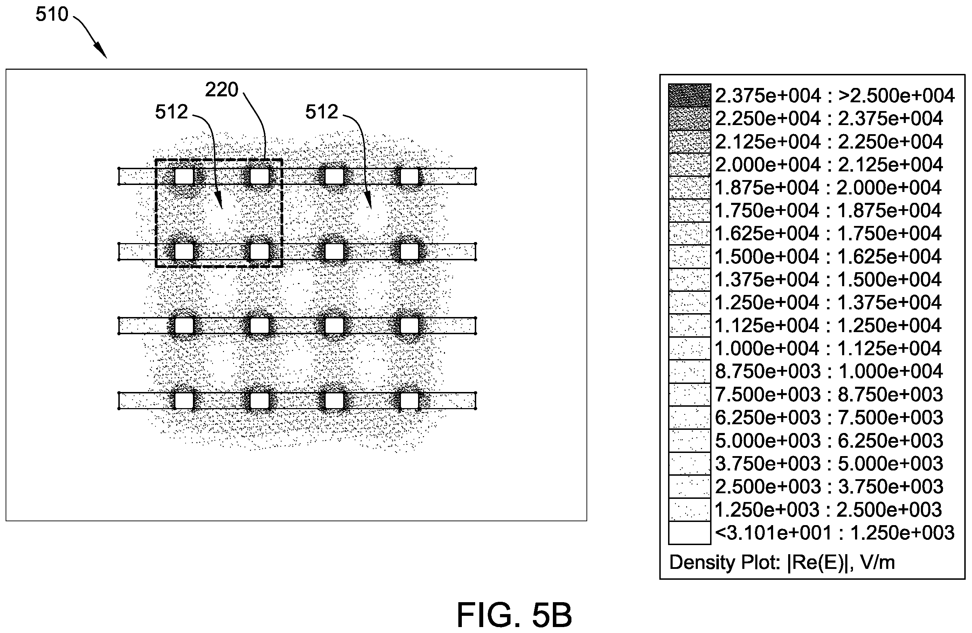

Figure 5B is a diagram 510 simulating an electric field strength (e.g., in Volts/centimeter (V/cm)) when energization pattern 502 is implemented. As shown in diagram 510, the electric field strength is highest around each electrode 210. In contrast, the electric field strength is low at low field spots 512. Low field spots 512 are generally located at a midpoint between adjacent electrodes 210 having the same polarity. Accordingly, with energization pattern 502, low field spots 512 occur at approximately the center of each quadripolar array 220. The low electric field strength occurs because the gradient of the electric field is at or near zero at low field spots 512.

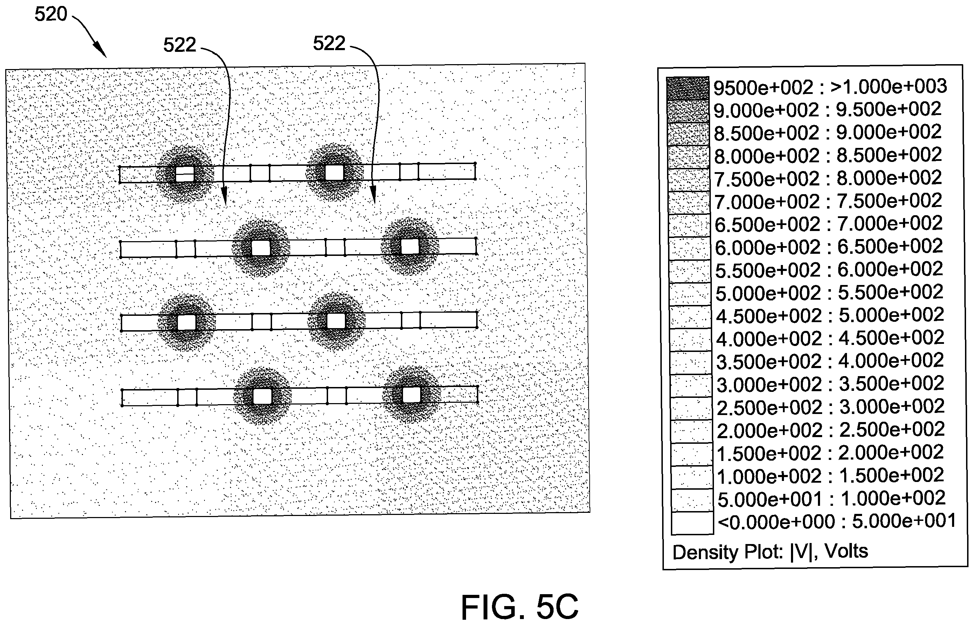

Figure 5C is a diagram 520 simulating the potential field when energization pattern 502 is implemented. As shown in Figure 5C, saddle points 522 correspond to the location of low field spots 512 in diagram 510. At saddle points 522, there is no slope, and thus no gradient (i.e., corresponding to zero electric field strength).

Notably, different energization patterns generally result in different low field spots. For example, Figure 6 is a diagram 600 simulating an electric field strength for an energization pattern that corresponds to using second energization pattern 404 (shown in Figure 4B) for each quadripolar array 220. Again, the electric field strength is highest around each electrode 210. However, in diagram 600, low field spots 602 occur between electrodes 210 that are located in the same row. Accordingly, low field spots 602 are located at different positions than low field spots 512 (shown in Figure 5B). Further, in diagram 600, at the locations corresponding to low field spots 512 from diagram 510, the electric field strength is relatively high.

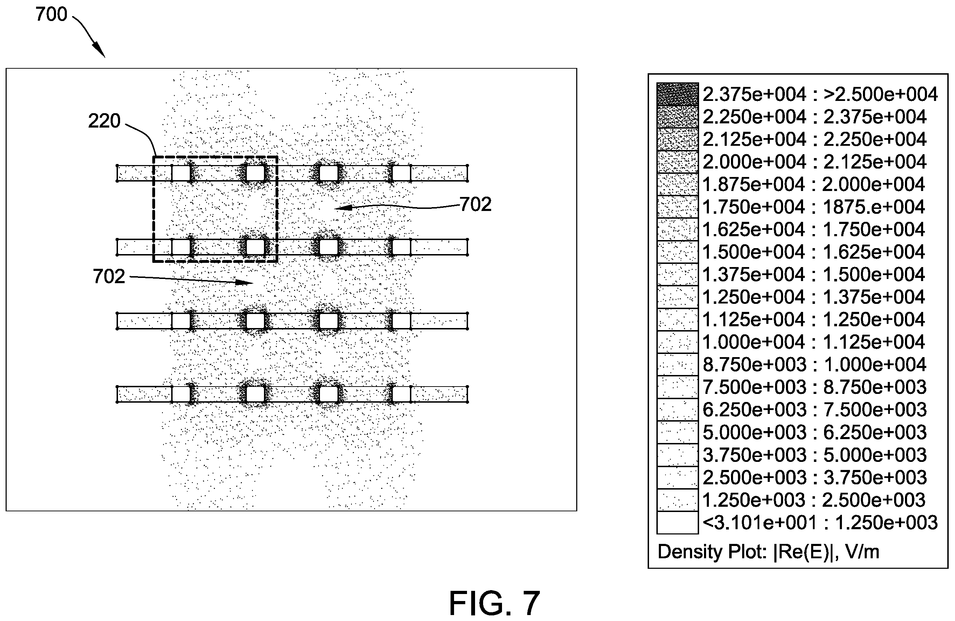

As another example, Figure 7 is a diagram 700 simulating an electric field strength for an energization pattern that corresponds to using third energization pattern 406 (shown in Figure 4C) for each quadripolar array 220. In diagram 700, low field spots 702 occur between electrodes 210 that are located in the same column. Again, low field spots 702 are located at different positions that low field spots 512 (shown in Figure 5B) and low field spots 602 (shown in Figure 6). Further, in diagram 700, at the locations corresponding to low field spots 512 from diagram 510 and the locations corresponding to low field spots 602 from diagram 600, the electric field strength is relatively high.

Accordingly, by applying combinations of energization patterns, a relatively uniform electric field strength can be achieved (as the low field spots in a particular energization pattern will be compensated for in other energization patterns). Thus, by cycling through multiple energization patterns, the overall ablation area generated will be relatively uniform.

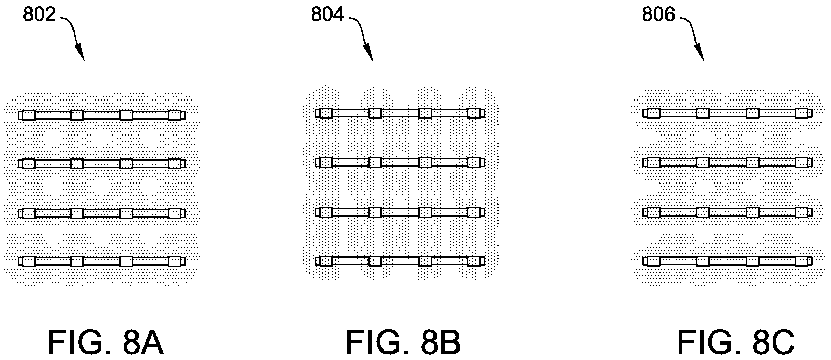

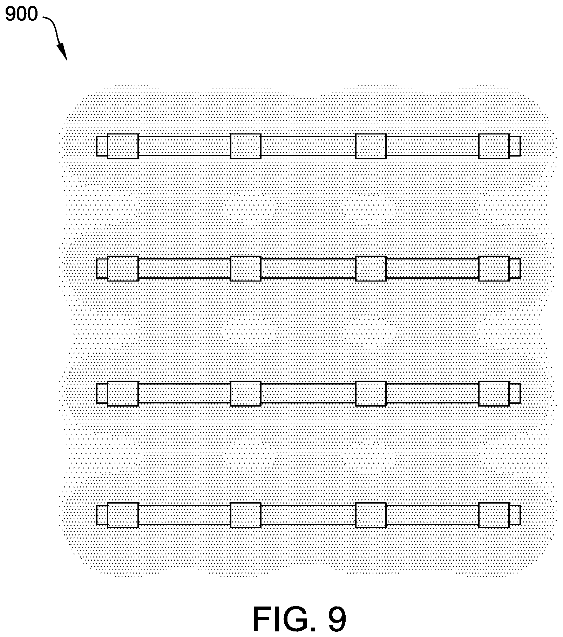

For example, Figure 8A is a representation 802 of diagram 510 (shown in Figure 5B), Figure 8B is a representation 804 of diagram 600 (shown in Figure 6), and Figure 8C is a representation 806 of diagram 700 (shown in Figure 7). Figure 9 is a diagram 900 showing representations 802, 804, and 806 superimposed on one another. As demonstrated by diagram 900, when representations 802, 804, and 806 are superimposed on one another (corresponds to cycling through all three energization patterns), the ablation area generated is relatively uniform, with holes in one energization pattern being filled by other energization patterns.

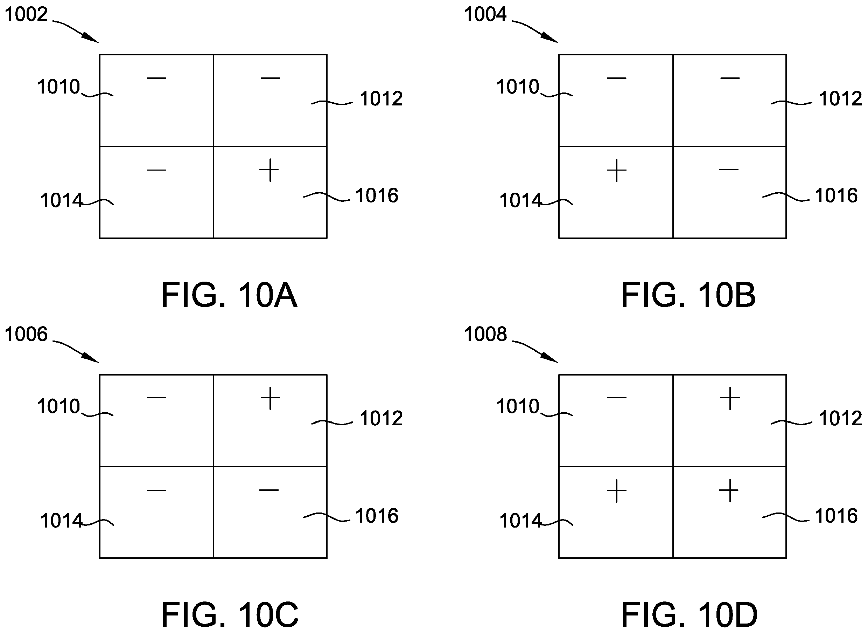

Those of skill in the art will appreciate that other energization patterns (i.e., other than those shown in Figures 4A-4C) may be used for each quadripolar array 220. For example, Figures 10A-10D illustrate a fourth energization pattern 1002, a fifth energization pattern 1004, a sixth energization pattern 1006, and a seventh energization pattern 1008. These energization patterns 1002, 1004, 1006, and 1008 are unbalanced (i.e., with an unequal number of positive and negative electrodes).

In fourth energization pattern 1002, a first electrode 1010 is negative, a second electrode 1012 is negative, a third electrode 1014 is negative, and a fourth electrode 1016 is positive. In fifth energization pattern 1004, first electrode 1010 is negative, second electrode 1012 is negative, third electrode 1014 is positive, and fourth electrode 1016 is negative. In sixth energization pattern 1006, first electrode 1010 is negative, second electrode 1012 is positive, third electrode 1014 is negative, and fourth electrode 1016 is negative. In seventh energization pattern 1008, first electrode 1010 is negative, second electrode 1012 is positive, third electrode 1014 is positive, and fourth electrode 1016 is positive.

Although the embodiments described herein are discussed in the context of IRE/PFA, those of skill in the art will appreciate that the methods and systems described herein may also be utilized for RF ablation applications.

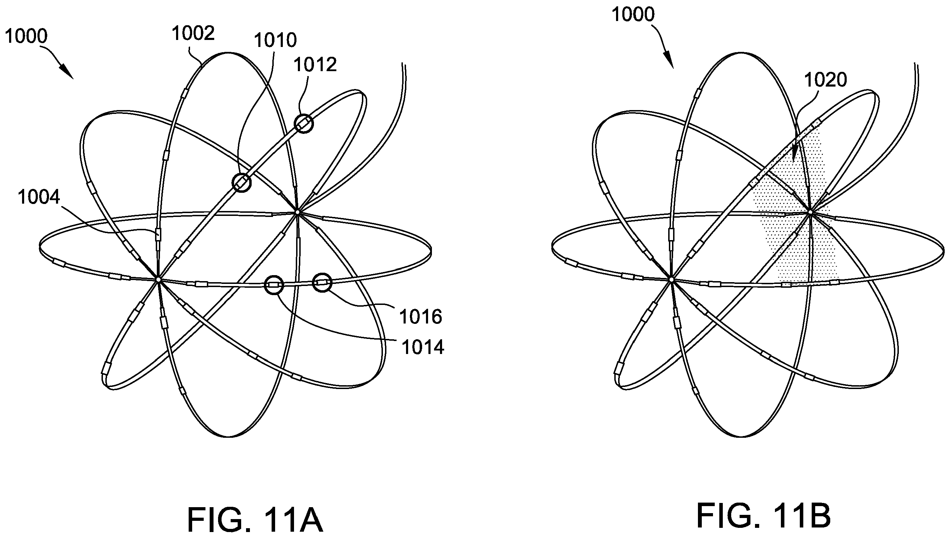

Further, those of skill in the art will appreciate that the techniques described herein may be implemented with catheter configurations other than grid assembly 200. For example, Figures 11A and 11B are perspective views of one embodiment of a basket assembly 1100 including a plurality of splines 1102 that form a basket, each spline including a plurality of electrodes 1104. Similar to grid assembly 200, quadripolar arrays can be defined by sets of electrodes 1104. For example, a first electrode 1110, second electrode 1112, third electrode 1114, and fourth electrode 1116 define a quadripolar array 1120 (shown in Figure 11B). Other catheter configurations may also utilize similar implementations.

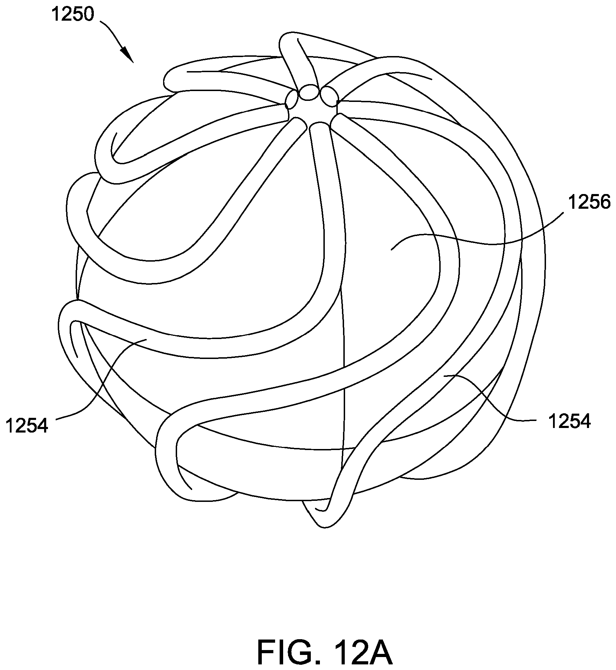

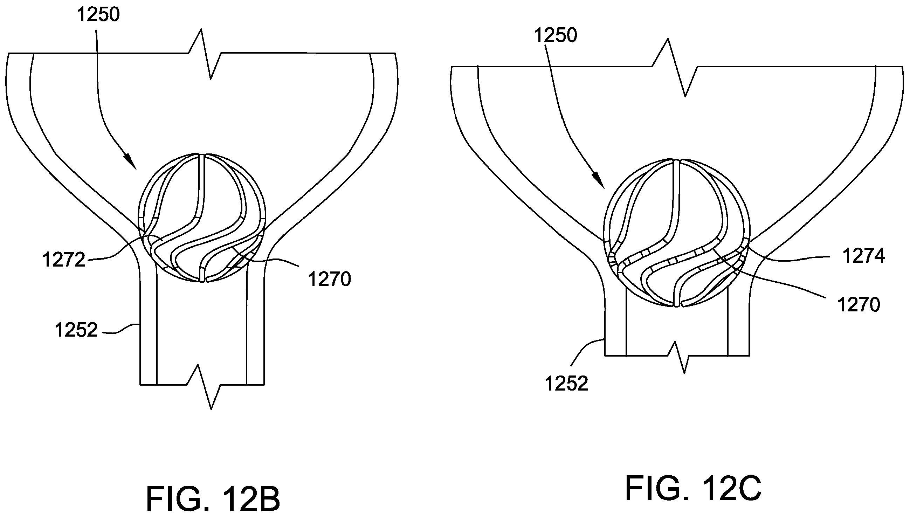

Figures 12A-12C are views of another embodiment of a basket assembly 1250 that may be used with the electrode energization techniques described herein. Specifically, Figure 12A is a perspective view of basket assembly 1250, and Figures 12B and 12C are side views of basket assembly 1250 positioned within a pulmonary vein 1252.

Basket assembly 1250 includes a plurality of splines 1254 that form a basket. In this embodiment, each spline 1254 has a generally sigmoidal shape. The sigmoidal shape of splines 1254 results in adjacent splines 1254 maintaining roughly the same distance between one another along the length of splines 1254, which may improve lesion quality. In this embodiment, basket assembly 1250 includes eight splines 1254. Alternatively, basket assembly 1250 may include any suitable number of splines 1254.

As shown in Figure 12A, basket assembly 1250 may include a selectively inflatable balloon 1256 positioned in an interior of the basket. Balloon 1256 may facilitate supporting splines 1254 (e.g., when splines are pressed against tissue). In some embodiments, balloon 1256 is omitted. Additional detail regarding basket assemblies with sigmoidal-shaped splines may be found in International Application No. PCT/US20/36410 entitled ELECTRODE BASKET HAVING HIGH-DENSITY CIRCUMFERENTIAL BAND OF ELECTRODES, filed on June 5, 2020, and U.S. Provisional Patent Application No. 62/861,135, entitled ELECTRODE BASKET HAVING HIGH-DENSITY CIRCUMFERENTIAL BAND OF ELECTRODES, filed on June 13, 2019.

Each spline 1254 include at least one electrode 1270 that is selectively energizable using the systems and methods disclosed herein. For example, Figure 7B shows one elongated electrode 1272 on each spline 1254, whereas Figure 7C shows a plurality of individual electrodes 1274 on each spline 1254. Electrodes 1270 are generally located on a distal portion of basket assembly 1250, to facilitate contacting tissue of pulmonary vein 1252. Alternatively, any suitable configuration of electrodes 1270 may be used. Similar to the embodiments described previously, sets of individual electrodes 1274 on basket assembly 1250 may define quadripolar arrays, and energization schemes similar to those described above may be suitably implemented.

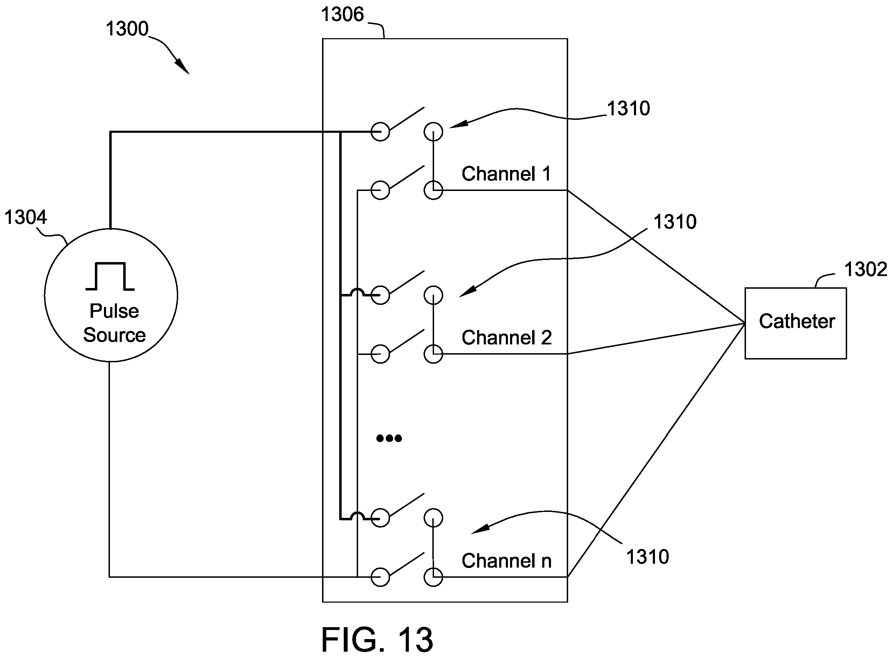

As described herein, electrodes on a catheter are selectively energized to generate different patterns. Figure 13 is a schematic diagram of one embodiment of a switching architecture 1300 that may be used to selectively energize electrodes on a catheter 1302. Specifically, switching architecture includes a catheter 1302, a pulse source 1304, and a switching unit 1306 coupled between catheter 1302 and pulse source 1304.

Pulse source 1304 generates energy pulses to be applied by the electrodes (not shown) on catheter 1302. Further, switching unit 1306 includes a plurality of switching circuits 1310 for selectively delivering energy pulses from pulse source 1304 to the electrodes. In this embodiment, switching unit 1306 includes a switching circuit 1310 (and corresponding channel) for each electrode. Each switching circuit 1310 receives an energy pulse from pulse source 1304 and, depending on a configuration of switches within switching circuit 1310, delivers a positive pulse, a negative pulse, or no pulse to the corresponding electrode. Accordingly, by controlling switching circuits 1310, the electrodes on catheter 1302 are selectively energizable.

The embodiments described herein are directed to an apparatus for controlling an electroporation catheter. The electroporation catheter includes a distal end, a proximal end, a plurality of splines extending from the distal end to the proximal end, and a plurality of electrodes arranged on the plurality of splines and defining at least one quadripolar array, each quadripolar array defined by four electrodes of the plurality of electrodes. The apparatus includes a pulse generator coupled to the electroporation catheter, and a computing device coupled to the pulse generator, the computing device operable to control the pulse generator to selectively energize the electrodes defining the at least one quadripolar array according to a first energization pattern, and selectively energize the electrodes defining the at least one quadripolar array according to a second energization pattern, wherein the first and second energization patterns are different from one another.

Although certain embodiments of this disclosure have been described above with a certain degree of particularity, those skilled in the art could make numerous alterations to the disclosed embodiments. All directional references (e.g., upper, lower, upward, downward, left, right. leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made within the scope of the invention as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

## Claims

1. An apparatus for controlling an electroporation catheter (14), the electroporation catheter including a distal end (48), a proximal end (46), a plurality of splines (204, 1102, 1254) extending from the distal end to the proximal end, and a plurality of electrodes (210, 410-416, 1010-1016, 1104, 1270) arranged on the plurality of splines and defining at least one quadripolar array (220, 1120), each quadripolar array defined by four electrodes of the plurality of electrodes, the apparatus comprising:
a pulse generator (26) coupled to the electroporation catheter; and
a computing device (32) coupled to the pulse generator, the computing device operable to control the pulse generator to:
selectively energize the electrodes defining the at least one quadripolar array according to a first energization pattern (402, 404, 406, 502, 1002, 1004, 1006, 1008); and
selectively energize the electrodes defining the at least one quadripolar array according to a second energization pattern (402, 404, 406, 502, 1002, 1004, 1006, 1008), wherein the first and second energization patterns are different from one another;
wherein energizing the electrodes according to the first energization pattern generates first low field spots (512, 522, 602, 702), wherein energizing the electrodes according to the second energization pattern generates second low field spots (512, 522, 602, 702), and wherein the first and second low field spots are located in different locations.

2. The apparatus in accordance with claim 1, wherein, in the first energization pattern (402, 404, 406, 502) and the second energization pattern (402, 404, 406, 502), each quadripolar array (220, 1120) includes two positive electrodes and two negative electrodes.

3. The apparatus in accordance with claim 1, wherein, in the first energization pattern (1002, 1004, 1006, 1008) and the second energization pattern (1002, 1004, 1006, 1008), each quadripolar array (220, 1120) includes a different number of positive electrodes and negative electrodes.

4. The apparatus in accordance with claim 1, wherein the computing device (32) is further operable to control the pulse generator (26) to:
selectively energize the electrodes defining the at least one quadripolar array (220, 1120) according to a third energization pattern, the third energization pattern different from the first and second energization patterns (402, 404, 406, 502, 1002, 1004, 1006, 1008).

5. The apparatus in accordance with claim 1, wherein the electroporation catheter (14) includes a grid assembly formed by the plurality of splines (204, 1102, 1254) and the plurality of electrodes, the computing device (32) operable to control the pulse generator (26) to selectively energize the plurality of electrodes on the grid assembly.

6. The apparatus in accordance with claim 1, wherein the electroporation catheter (14) includes a basket assembly formed by the plurality of splines (204, 1102, 1254) and the plurality of electrodes, the computing device (32) operable to control the pulse generator (26) to selectively energize the plurality of electrodes on the basket assembly.

7. The apparatus in accordance with claim 1, wherein the computing device (32) is operable to control the pulse generator (26) to selectively energize the plurality of electrodes to delivery bipolar therapy.

8. A system comprising:
an electroporation catheter (14) comprising a distal end (48), a proximal end (46), a plurality of splines (204, 1102, 1254) extending from the distal end to the proximal end, and a plurality of electrodes (210, 410-416, 1010-1016, 1104, 1270) arranged on the plurality of splines and defining at least one quadripolar array (220, 1120), each quadripolar array defined by four electrodes of the plurality of electrodes; and
an apparatus for controlling the electroporation catheter according to any of claims 1 to 7.

## Patentansprüche

1. Eine Vorrichtung zur Steuerung eines Elektroporationskatheters (14), wobei der Elektroporationskatheter ein distales Ende (48), ein proximales Ende (46), eine Vielzahl von Rippen (204, 1102, 1254), die sich von dem distalen Ende zu dem proximalen Ende erstrecken, und eine Vielzahl von Elektroden (210, 410-416, 1010-1016, 1104, 1270) einschließt, die auf der Vielzahl von Rippen angeordnet sind und mindestens ein quadripolares Array (220, 1120) definieren, wobei jedes quadripolare Array durch vier Elektroden der Vielzahl von Elektroden definiert wird, wobei die Vorrichtung Folgendes umfasst:
einen Impulsgenerator (26), der mit dem Elektroporationskatheter gekoppelt ist; und
ein Computergerät (32), das mit dem Impulsgenerator gekoppelt ist, wobei das Computergerät betriebsfähig ist, um den Impulsgenerator für Folgendes zu steuern:
selektives Erregen der Elektroden, die das mindestens eine quadripolare Array definieren, nach einem ersten Erregungsmuster (402, 404, 406, 502, 1002, 1004, 1006, 1008); und
selektives Erregen der Elektroden, die das mindestens eine quadripolare Array definieren, nach einem zweiten Erregungsmuster (402, 404, 406, 502, 1002, 1004, 1006, 1008), wobei das erste und zweite Erregungsmuster sich voneinander unterscheiden;
wobei das Erregen der Elektroden nach dem ersten Erregungsmuster erste Punkte mit geringem Feld (512, 522, 602, 702) erzeugt, wobei das Erregen der Elektroden nach dem zweiten Erregungsmuster zweite Punkte mit geringem Feld (512, 522, 602, 702) erzeugt, und wobei die ersten und zweiten Punkte mit geringem Feld sich an verschiedenen Orten befinden.

2. Vorrichtung nach Anspruch **1,** wobei jedes quadripolare Array (220, 1120) in dem ersten Erregungsmuster (402, 404, 406, 502) und dem zweiten Erregungsmuster (402, 404, 406, 502) zwei positive Elektroden und zwei negative Elektroden einschließt.

3. Vorrichtung nach Anspruch **1,** wobei jedes quadripolare Array (220, 1120) in dem ersten Erregungsmuster (1002, 1004, 1006, 1008) und dem zweiten Erregungsmuster (1002, 1004, 1006, 1008) eine verschiedene Anzahl positiver Elektroden und negativer Elektroden einschließt.

4. Vorrichtung nach Anspruch **1,** wobei das Computergerät (32) ferner betriebsfähig ist, den Impulsgenerator (26) für Folgendes zu steuern:
selektives Erregen der Elektroden, die das mindestens eine quadripolare Array (220, 1120) definieren, nach einem dritten Erregungsmuster, wobei das dritte Erregungsmuster sich von dem ersten und zweiten Erregungsmuster (402, 404, 406, 502, 1002, 1004, 1006, 1008) unterscheidet.

5. Vorrichtung nach Anspruch **1,** wobei der Elektroporationskatheter (14) eine Gitteranordnung einschließt, die durch die Vielzahl von Rippen (204, 1102, 1254) und die Vielzahl von Elektroden gebildet wird, wobei das Computergerät (32) betriebsfähig ist, um den Impulsgenerator (26) zu steuern, um die Vielzahl von Elektroden auf der Gitteranordnung selektiv zu erregen.

6. Vorrichtung nach Anspruch **1,** wobei der Elektroporationskatheter (14) eine Korbanordnung einschließt, die durch die Vielzahl von Rippen (204, 1102, 1254) und die Vielzahl von Elektroden gebildet wird, wobei das Computergerät (32) betriebsfähig ist, um den Impulsgenerator (26) zu steuern, um die Vielzahl von Elektroden auf der Korbanordnung selektiv zu erregen.

7. Vorrichtung nach Anspruch **1,** wobei das Computergerät (32) betriebsfähig ist, um den Impulsgenerator (26) zu steuern, um die Vielzahl von Elektroden selektiv zu erregen, um bipolare Therapie zuzuführen.

8. Ein System, das Folgendes umfasst:
einen Elektroporationskatheter (14), der ein distales Ende (48), ein proximales Ende (46), eine Vielzahl von Rippen (204, 1102, 1254), die sich von dem distalen Ende zu dem proximalen Ende erstrecken, und eine Vielzahl von Elektroden (210, 410-416, 1010-1016, 1104, 1270) umfasst, die auf der Vielzahl von Rippen angeordnet sind und mindestens ein quadripolares Array (220, 1120) definieren, wobei jedes quadripolare Array durch vier Elektroden der Vielzahl von Elektroden definiert wird; und
eine Vorrichtung zur Steuerung des Elektroporationskatheters nach einem der Ansprüche 1 bis 7.

## Revendications

1. Appareil destiné à commander un cathéter d'électroporation (14), le cathéter d'électroporation incluant une extrémité distale (48), une extrémité proximale (46), une pluralité de cannelures (204, 1102, 1254) s'étendant de l'extrémité distale à l'extrémité proximale, et une pluralité d'électrodes (210, 410-416, 1010-1016, 1104, 1270) agencées sur la pluralité de cannelures et définissant au moins un réseau quadripolaire (220, 1120), chaque réseau quadripolaire étant défini par quatre électrodes de la pluralité d'électrodes, l'appareil comprenant :
un générateur d'impulsions (26) couplé au cathéter d'électroporation ; et
un dispositif d'ordinateur (32) couplé au générateur d'impulsions, le dispositif de calcul capable de fonctionner pour commander le générateur d'impulsions pour :
exciter sélectivement les électrodes définissant l'au moins un réseau quadripolaire selon un premier schéma d'excitation (402, 404, 406, 502, 1002, 1004, 1006, 1008) ; et
exciter sélectivement les électrodes définissant l'au moins un réseau quadripolaire selon un deuxième schéma d'excitation (402, 404, 406, 502, 1002, 1004, 1006, 1008), dans lequel les premier et deuxième schémas d'excitation sont différents l'un de l'autre ;
dans lequel l'excitation des électrodes selon le premier schéma d'excitation génère des premiers points de faible champ (512, 522, 602, 702), dans lequel l'excitation des électrodes selon le deuxième schéma d'excitation génère des deuxièmes points de faible champ (512, 522, 602, 702), et dans lequel les premiers et deuxièmes points de faible champ sont situés dans des emplacements différents.

2. Appareil selon la revendication 1, dans lequel, dans le premier schéma d'excitation (402, 404, 406, 502) et le deuxième schéma d'excitation (402, 404, 406, 502), chaque réseau quadripolaire (220, 1120) inclut deux électrodes positives et deux électrodes négatives.

3. Appareil selon la revendication 1, dans lequel, dans le premier schéma d'excitation (1002, 1004, 1006, 1008) et le deuxième schéma d'excitation (1002, 1004, 1006, 1008), chaque réseau quadripolaire (220, 1120) inclut un nombre différent d'électrodes positives et d'électrodes négatives.

4. Appareil selon la revendication 1, dans lequel le dispositif de calcul (32) est en outre capable de fonctionner pour commander le générateur d'impulsions (26) pour :
exciter sélectivement les électrodes définissant l'au moins un réseau quadripolaire (220, 1120) selon un troisième schéma d'excitation, le troisième schéma d'excitation étant différent des premier et deuxième schémas d'excitation (402, 404, 406, 502, 1002, 1004, 1006, 1008).

5. Appareil selon la revendication 1, dans lequel le cathéter d'électroporation (14) inclut un ensemble de grille formé par la pluralité de cannelures (204, 1102, 1254) et la pluralité d'électrodes, le dispositif de calcul (32) capable de fonctionner pour commander le générateur d'impulsions (26) pour exciter sélectivement la pluralité d'électrodes sur l'ensemble de grille.

6. Appareil selon la revendication 1, dans lequel le cathéter d'électroporation (14) inclut un ensemble de panier formé par la pluralité de cannelures (204, 1102, 1254) et la pluralité d'électrodes, le dispositif de calcul (32) capable de fonctionner pour commander le générateur d'impulsions (26) pour exciter sélectivement la pluralité d'électrodes sur l'ensemble de panier.

7. Appareil selon la revendication 1, dans lequel le dispositif de calcul (32) est capable de fonctionner pour commander le générateur d'impulsions (26) pour exciter sélectivement la pluralité d'électrodes pour délivrer une thérapie bipolaire.

8. Système (100) comprenant :
un cathéter d'électroporation (14), comprenant une extrémité distale (48), une extrémité proximale (46), une pluralité de cannelures (204, 1102, 1254) s'étendant de l'extrémité distale à l'extrémité proximale, et une pluralité d'électrodes (210, 410-416, 1010-1016, 1104, 1270) agencées sur la pluralité de cannelures et définissant au moins un réseau quadripolaire (220, 1120), chaque réseau quadripolaire défini par quatre électrodes de la pluralité d'électrodes ; et
un appareil destiné à commander le cathéter d'électroporation selon l'une quelconque des revendications 1 à 7.
